# EUROPEAN PATENT APPLICATION

(11) **EP 3 431 054 A1**
(43) Date of publication of application: **23.01.2019**
(21) Application number: 17182450.1
(22) Date of filing: 20.07.2017
(51) Int. Cl.: A61F 5/01

(54) **HINGE ASSEMBLY**

(71) Applicant: Peacocks Medical Group Ltd, Newcastle upon Tyne Tyne and Wear NE6 4NQ (GB)
(72) Inventor: DESSERY, Yoann, Newcastle upon Tyne, Tyne and Wear NE6 4NQ (GB); ESKANDARI, Behrokh, Newcastle upon Tyne, Tyne and Wear NE6 4NQ (GB); PALLARI, Jari, Newcastle upon Tyne, Tyne and Wear NE6 4NQ (GB)
(74) Representative: Retter, Jocelyn Anna

(57) **Abstract**

A hinge assembly for a brace (10) for supporting or guiding movement of a joint of a limb is described. The hinge assembly comprises: a leaf spring hinge (16) comprising a plurality of spring leaves (60), wherein each spring leaf (60) comprises first and second end portions (64, 66) and a middle portion (68) extending longitudinally therebetween; and a first socket assembly (20) comprising a recess (22, 32) for receiving respective first end portions (64) of the spring leaves (60; 60a) such that the middle portion (68) of at least one said spring leaf (60; 60a) extends through an opening (24; 34) in communication with said recess (22; 32); wherein the first end portion (64; 64a) of said at least one spring leaf (60 60a) has a transverse dimension (X2; Y2) greater than a corresponding transverse dimension (X1; Y1) of the middle portion (68) of the spring leaf (60; 60a), and wherein said opening (24; 34) has a transverse dimension (X3; Y3) which is smaller than the corresponding transverse dimension (X2; Y2) of said first end portion (64; 64a) of said at least one spring leaf (60; 60a).

## Description

### Field of the Invention

The present invention relates to a hinge assembly for a brace for supporting or guiding movement of a joint of a limb. In particular, but not exclusively, the present invention relates to a hinge assembly for a knee brace comprising a leaf spring.

### Background

Unloader knee braces are medical devices mainly used in the management of knee osteoarthritis. These devices aim to relieve pain, decrease symptoms and unload the affected compartment of the knee by correcting the misalignment of the lower limb. One type of unloader knee brace comprises two semi-rigid or hard shells, to be worn around the thigh and shank respectively, connected by a unilateral hinge or bilateral hinges. Correction of the misalignment can be achieved using three major mechanisms: pushing, pulling or rotation and distraction. For the pushing and pulling systems, the amount of correction can be modified by bending the knee brace or tightening a diagonal strap extending between upper and lower parts of the brace, thereby applying a greater force towards the direction desired.

The hinge or hinges aim to follow the knee joint motion path during flexion and extension of the knee. Various hinges are currently used, including simple pivot, aligned polycentric, or misaligned polycentric hinges. However, knee joint motion is a combination of gliding and sliding, resulting in a moving centre of rotation. Because each knee is unique, no existing hinge for unloader knee braces perfectly manages the change in the centre of rotation, so users experience discomfort and migration of the knee brace. This is one of the reasons why around 42% of users stop wearing a knee brace in the first year of use.

A leaf spring hinge has also been used in a soft knee brace for stabilising the knee. The leaf spring hinge does not have a fixed sense of rotation and so follows the knee joint motion path in a better way, while limiting the adduction/abduction movement of the knee. In this soft knee brace, each end of the leaf spring hinge is inserted into a plastic housing directly sewn on a fabric sleeve worn around the knee, while another plastic housing in the middle of the hinge keeps the layers of the leaf spring hinge together.

Embodiments of the present invention seek to overcome disadvantages associated with the prior art.

### Statements of Invention

According to a first aspect of the present invention, there is provided a hinge assembly for a brace for supporting or guiding movement of a joint of a limb, the hinge assembly comprising:
a leaf spring hinge comprising a plurality of spring leaves, wherein each spring leaf comprises first and second end portions and a middle portion extending longitudinally therebetween; and
a first socket assembly comprising a recess for receiving respective first end portions of the spring leaves such that the middle portion of at least one said spring leaf extends through an opening in communication with said recess;
wherein the first end portion of at said at least one spring leaf has a transverse dimension greater than a corresponding transverse dimension of the middle portion of the spring leaf, and wherein said opening has a transverse dimension which is smaller than the corresponding transverse dimension of said first end portion of said at least one spring leaf.

Advantageously, when the hinge assembly is assembled, the or each spring leaf is retained by the socket assembly, preventing the hinge assembly from being pulled apart. This is particularly important when the hinge assembly is incorporated into a hard brace such as an unloader brace, in which the upper and lower parts of the brace must be held together by the hinge. The invention thereby enables a leaf spring hinge to be used in a hard brace, without a fixed-pivot connection between the upper and lower parts of the brace. This provides the advantage that, unlike the fixed-pivot hinges conventionally used with hard braces, the leaf spring hinge does not constrain the centre of rotation of the joint but instead allows the joint to flex more naturally. The leaf spring hinge does not have a fixed centre of rotation, so adapts to the change in centre of rotation of a user's joint.

Said first end portion of said at least one spring leaf may comprise an end cap attached to said spring leaf.

The leaf spring hinge may comprise one or more friction-reducing leaves inserted between at least one respective pair of adjacent said spring leaves.

The socket assembly may comprise a constraining element for constraining longitudinal movement of the end portion of an outermost one of the plurality of spring leaves relative to the socket assembly with respect to the other spring leaves.

By preventing or constraining longitudinal movement of the end portion of an outermost one of the plurality of spring leaves relative to the socket assembly, the distance between the upper and lower parts of a brace incorporating the hinge assembly may be fixed or constrained between predetermined limits, while enabling the other leaves of the leaf spring hinge to slide relative to each other as required for normal functioning of the leaf spring hinge.

The constraining element may be arranged to prevent longitudinal movement of the end portion of the outermost one of the plurality of spring leaves relative to the socket assembly.

This feature enables the distance between upper and lower parts of a brace incorporating the hinge assembly to be fixed.

Said constraining element may comprise a channel defined between two internal surfaces of said socket assembly and arranged to receive the middle portion of said outermost one of said plurality of spring leaves, wherein said channel has a transverse dimension smaller than a corresponding transverse dimension of said first end portion of said outermost spring leaf.

In some embodiments, said constraining element, including said channel, is provided in addition to said recess and said opening defined above. In other embodiments, said opening is provided by said channel.

The outermost spring leaf may be longer than the other spring leaves.

This feature enables the constraining element for securing the outermost spring leaf to be located beyond the position of the other spring leaves, in a longitudinal direction.

The first socket assembly may comprise a first socket part, fixable to or integrated into a part of the brace for attachment to the limb of a user, and a second socket part removably fixable to said first socket part, wherein said end portion of said at least one spring leaf is receivable between opposing surfaces of said first and second socket parts.

This feature facilitates assembly of the hinge assembly and enables release of the leaf spring from the socket assembly for disassembly and/or adjustment.

The second socket part may be receivable in said first socket part.

This feature may improve the ease of assembly of the hinge assembly. For example, it may enable the spring leaves to be inserted with ease into the first socket part prior to insertion of the second socket part into the first socket part. A further advantage of this feature is that it may enable the first and second socket parts to be fixed to each other by fastening elements (e.g. screws) accessed from a limb-facing surface of a brace in which the hinge assembly is comprised, thereby improving the outward appearance of the brace and preventing fastening elements from snagging on clothing or causing obstruction during use of the brace.

Said channel may be defined between two surfaces of said second socket part.

This feature may also improve ease of assembly of the hinge assembly, by allowing the outermost spring leaf to be correctly located in the channel on the second socket part, prior to insertion of the second socket part into the first socket part and/or after insertion of the spring leaves into the first socket part.

Said socket assembly may comprise a wedge portion for adjusting a lateral angle of a brace part attached to or integrated to said socket assembly relative to said leaf spring.

Advantageously, this feature enables adjustment of a lateral angle between first and second brace parts connected by the hinge assembly by a predetermined angle. The hinge assembly may perform a dual function of angle correction and restraint of the leaf spring.

Said wedge portion may be attached to or integrally formed with said second socket part.

This feature enables the lateral angle between the upper and lower brace parts to be adjusted by replacing the wedge portion or the second socket part, without replacing the entire hinge assembly or brace. Some existing knee braces include an adjustable angle correction, which can be incorrectly adjusted by patients to their detriment. By providing angle correction by integrally forming a wedge portion with the second socket part, the angle correction cannot easily be modified by a patient unless supplied with replacement parts.

The hinge assembly may further comprise:
a second socket assembly comprising a second recess for receiving respective second end portions of the spring leaves such that the middle portion of at least one said spring leaf extends through a second opening in communication with said second recess;
wherein the second end portion of said at least one spring leaf has a transverse dimension greater than a corresponding transverse dimension of the middle portion of the spring leaf, and wherein said second opening has a transverse dimension which is smaller than a corresponding transverse dimension of said second end portion of said at least one spring leaf.

Advantageously, when the hinge assembly is assembled, the first and second ends of the/or each spring leaf may both be retained by respective first and second socket assemblies, preventing the hinge assembly from being pulled apart. This is particularly important in a hard brace, in which the upper and lower parts of the brace must be held together by the hinge.

The first and/or second socket part may be formed by an additive manufacturing technique.

According to another aspect of the present invention, there is provided a brace for supporting or guiding movement of a joint of a limb, the brace comprising:
an upper brace part for attachment to the limb above the joint;
a lower brace part for attachment to the limb below the joint; and
a hinge assembly according to the first aspect, the hinge assembly connecting the upper and lower brace parts;
wherein said first socket assembly is fixed to one of said upper and lower brace parts.

Said second socket assembly may be fixed to the other one of said upper and lower brace parts.

The upper and/or lower brace part may comprise a hard shell.

The upper and/or lower brace part may be formed by an additive manufacturing technique.

This feature enables the upper and/or lower brace part to be readily adapted to a specific user's needs.

The brace may comprise a second hinge assembly connecting the upper and lower brace parts. The first hinge may be arranged on a first side of the brace and the second hinge assembly may be located on a second side of the brace, opposite to the first hinge assembly.

The brace may be a knee brace. The brace may be an unloader knee brace.

### Brief description of the drawings

Preferred embodiments of the invention will now be described, by way of example only and not in any limitative sense, with reference to the accompanying drawings, in which:
Figure 1 shows a knee brace comprising a hinge assembly according to an embodiment of the present invention;
Figure 2a is an exploded illustration of a leaf spring hinge of a hinge assembly according to an embodiment of the invention;
Figure 2b illustrates cross-sections of a spring leaf of the leaf spring hinge of Figure 2a;
Figure 3 is a cut-away view of a hinge assembly according to an embodiment of the present invention, shown as partially transparent to show its interior structure;
Figure 4a is an illustration of a socket assembly of the hinge assembly of Figure 3;
Figure 4b is a cutaway view of the socket assembly of Figure 4a;
Figure 4c is an exploded view of the socket assembly of Figure 4a;
Figure 4d is a perspective view of the socket assembly of Figure 4a;
Figures 4e, 4f, 4g and 4h show cutaway views of the socket assembly of Figure 4a;
Figure 5a and 5b are perspective views and Figure 5c is a cutaway view of a first socket part of the socket assembly of Figures 4a-h;
Figure 6 shows a second socket part of the socket assembly of Figures 4a-h;
Figures 7a, 7b and 7c illustrate a sequence of steps of a method for assembling the hinge assembly of Figure 3; and
Figure 8 illustrates a second socket part of a socket assembly of a hinge assembly according to a further embodiment of the invention.

### Detailed Description of Embodiments

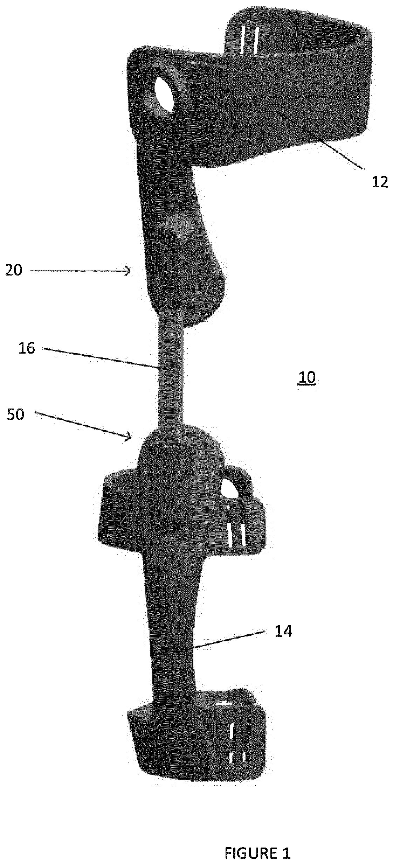

Figure 1 shows a brace 10, in the form of a knee brace 10, incorporating a hinge assembly according to an embodiment of the present invention. The brace 10 includes a first or upper brace part 12 for attachment to the thigh of a user and a second or lower brace part 14 for attachment to the shank of a user. The upper and lower brace parts 12, 14 are hard shells. The knee brace 10 is adapted to support or guide movement of the user's knee. The hinge assembly of the brace 10 includes a leaf spring hinge 16, a first socket assembly 20 provided on the upper brace part 12 and a second socket assembly 50 provided on the lower brace part 14. The first and second socket assemblies 20, 50 receive respective first and second ends of the leaf spring hinge 16. The brace 10 is arranged such that, in use, the hinge assembly is located laterally of the knee joint of a user. In alternative embodiments, a brace may include a second hinge assembly arranged such that, in use, the first and second hinge assemblies are located on opposite lateral sides of the user's joint. However, the present invention is not limited to braces with one or more lateral hinges. The hinge assembly of the present invention may be adapted for use in other positions within a brace.

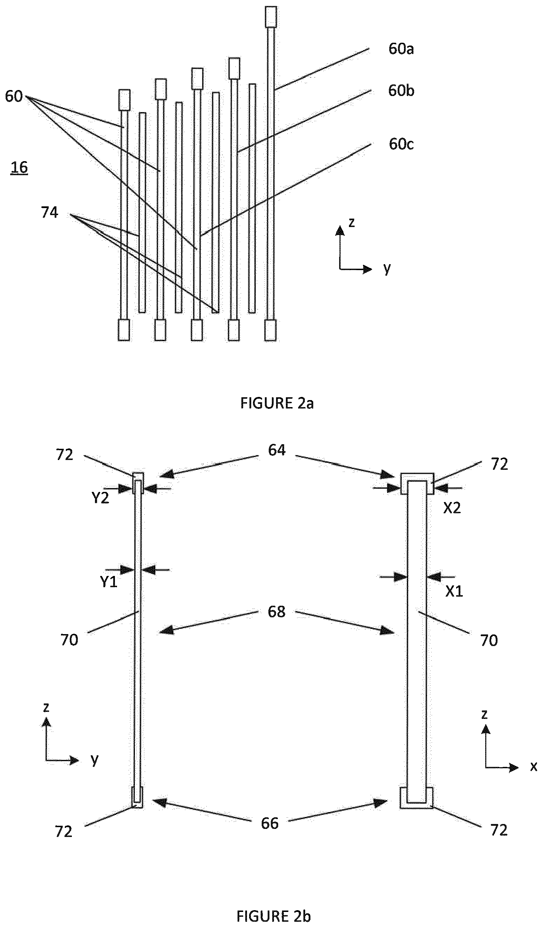

Figure 2a illustrates the leaf spring hinge 16, which comprises a plurality of individual spring leaves 60, interleaved with friction-reducing leaves 74. The total number of individual spring leaves 60, and their thicknesses, are selected depending on the required stiffness.

Figure 2b illustrates longitudinal cross-sections of a single spring leaf 60. For the purpose of this description, we define axes x, y, and z relative to the spring leaves 60. Each spring leaf 60 comprises a first end portion 64, a second end portion 66, and a middle portion 68 extending longitudinally (parallel to the z-axis) between the first and second end portions 64, 66. The middle portion 68 of each spring leaf 60 has a substantially rectangular transverse cross-section having transverse dimensions X1 (in the x-direction) and Y1 (in the y-direction), with X1 being greater than Y1. The end portions 64, 66 of each spring leaf 60 have a transverse cross-section which is larger than that of the middle portion 68. In this embodiment, the transverse cross-section of the end portions 64, 66 has dimensions X2, Y2, each of which are larger than the corresponding transverse dimension of the middle portion 68, i.e. both X2>X1 and Y2>Y1. However, in some embodiments, the end portions 64, 66 may be enlarged relative to the middle portion 68 in only one of the transverse directions, i.e. parallel to the x- or y-axis.

In the embodiment shown in Figure 2b, the spring leaf 60 comprises a spring leaf body 70, for example a steel spring leaf 70, and end caps 72, for example metal end caps 72, at each end of the spring leaf body 70. The end caps 72 are fixed to the ends of the spring leaf body 70, for example by crimping or gluing, resulting in the end portions 64, 66 having an enlarged cross-section relative to the spring leaf body 70.

The leaf spring hinge 16 bends about an axis parallel to the x-axis, with a stiffness dependent on the number of spring leaves stacked together to form the spring and the stiffness of the individual spring leaves 60. The stiffness of each individual spring leaf 60 depends in part on its thickness, in particular its thickness Y1 in the y direction. The axis about which the hinge 16 bends may shift as the hinge angle increases. The spring leaf hinge is relatively stiff with respect to bending about an axis parallel to the y-axis. A small amount of twisting is also possible about the z-axis, i.e. relative rotation about the z-axis between the first and second end portion 64, 66.

As the leaf spring hinge 16 bends, the individual spring leaves 60 slide relative to each other, to take account of their different radii of curvature when the hinge 16 is bent. In this embodiment, friction-reducing leaves 74, in the form of plastic leaves 74, are provided between neighbouring spring leaves 60, such that the spring leaves 60 and plastic leaves 74 are arranged in an alternating manner. The plastic leaves 74 reduce friction between neighbouring spring leaves 60, but may be omitted in other embodiments. The resulting leaf spring hinge 16 has a transverse dimension parallel to the x-axis corresponding to that of the individual spring leaves 60, i.e. X1, X2, and a transverse dimension parallel to the y-axis depending on the number of stacked spring leaves 60 and plastic leaves 74 forming the spring leaf hinge 16, together with their thicknesses in the y-direction.

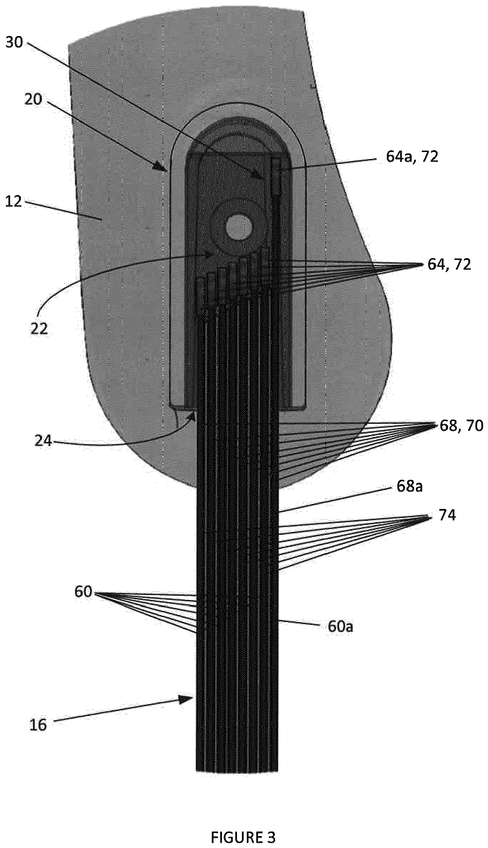

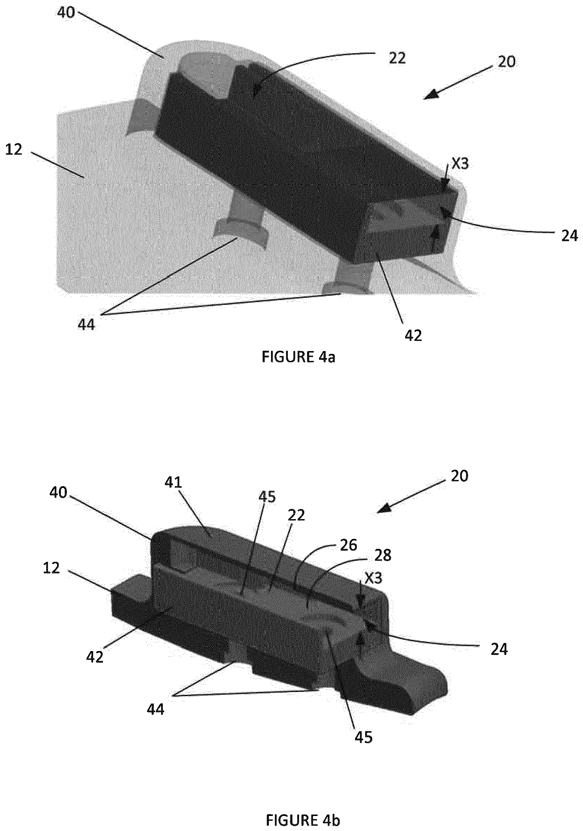

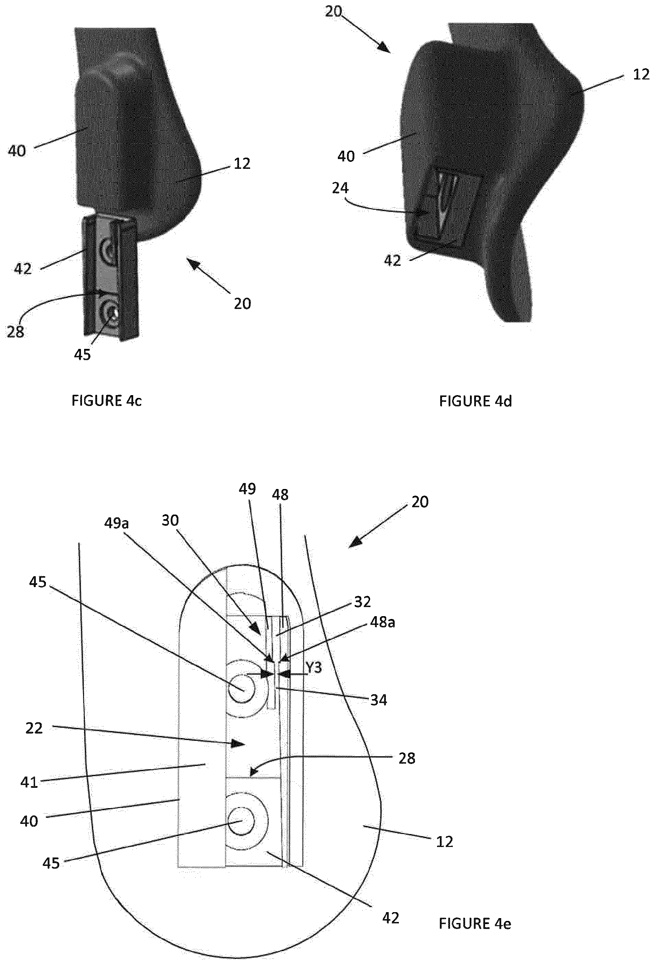

Figure 3 is a cutaway view of the hinge assembly, with the first end portions 64 of the spring leaves 60 of the leaf spring hinge 16 received in the first socket assembly 20. The second end portions 66 of the spring leaves 60 are received in the second socket assembly 50 (see Figure 1). Figures 4a-h illustrate the first socket assembly 20 in more detail. The second socket assembly 50 will not be described in detail, but includes features corresponding to those of the first socket assembly 20. With reference to Figures 3 and 4a-h, the socket assembly 20 includes a recess 22 which receives the first end portions 64 of the stacked spring leaves 60 such that the middle portions 68 of the spring leaves 60 extend out of the socket assembly 20 through an opening 24 in communication with the recess 22.

As explained above, the end portions 64 of the spring leaves have a transverse dimension X2 in the x-direction greater than a corresponding transverse dimension X1 of the middle portions 68 of the spring leaves 60. The recess 22 of the socket assembly 20 has a transverse dimension in the x-direction which is slightly greater than the corresponding transverse dimension X2 of the first end portions 64 of the spring leaves 60, thereby allowing the end portions 64 to slide longitudinally (along the z-direction) in the recess 22. The opening 24 of the recess 22 of the socket assembly 20 has a transverse dimension X3 which is smaller than the corresponding transverse dimension X2 of the end portions 64 of the spring leaves 60, but slightly larger than the corresponding transverse dimension X1 of the middle portions 68 of the spring leaves 60, allowing the middle portions 68 to slide longitudinally through the opening 24. The change in transverse size (in the x-direction) between the recess 22 and the opening 24 is achieved by providing steps 26, 28 in the opposing internal surfaces of the socket assembly 20. In the other transverse direction, i.e. parallel to the y-axis defined above, the leaf spring hinge 16 nearly fills the opening 24, to the extent that the individual spring leaves 60 are substantially unable to rotate relative to the socket assembly 20. Depending on the selected number of spring leaves 60 included in the leaf spring hinge 16, the width of the leaf spring hinge 16 in the y-direction may be increased if required by adding further friction-reducing or plastic leaves 74. As a result, the end portions 64 of the spring leaves 60 are trapped inside the recess 22 of the socket assembly 20. This feature prevents the upper and lower brace parts 12, 14 from being pulled apart from each other.

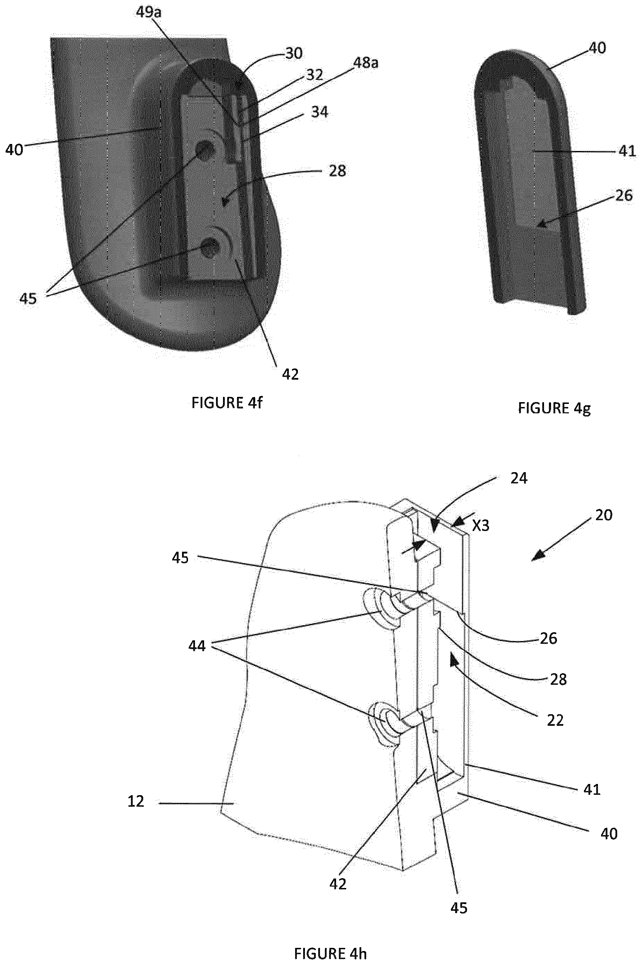

With particular reference to Figures 3, 4e and 4f, the socket assembly 20 also comprises a constraining element 30 for fixing or constraining longitudinal movement of the outermost one 60a of the plurality of spring leaves 60 relative to the socket assembly 20. In this embodiment, the constrained outermost spring leaf 60a is the one which has the greatest radius of curvature when the leaf spring hinge 16 is bent, i.e. when the hinge assembly is integrated into the knee brace 10, it is the forwardmost spring leaf relative to a user wearing the brace.

In this embodiment the constraining element 30 is located within the recess 22 of the socket assembly 20. The constraining element 30 comprises a further recess 32 and a further opening or channel 34 in communication with the further recess 32. The further opening or channel 34 is also in communication with the opening 24 of the socket assembly 20. The further recess 32 is configured to receive the end portion 64a of the outermost spring leaf 60a such that the middle portion 68a of the outermost spring leaf 60a extends through the further opening or channel 34 and through the opening 24.

As explained above, the end portions 64 of the spring leaves have a transverse dimension Y2 in the y-direction greater than a corresponding transverse dimension Y1 of the middle portion 68 of the spring leaf 60. The further opening or channel 34 of the recess 32 of the socket assembly 20 has a transverse dimension Y3 in the y-direction which is smaller than the corresponding transverse dimension Y2 of the end portion 64a of the outermost spring leaf 60a, but slightly larger than (or closely matched to) the corresponding transverse dimension Y1 of the middle portion 68a of the outermost spring leaf 60a. As a result, the end portion 64a of the outermost spring leaf 60a is trapped inside the recess 32 of the constraining element 30 of the socket assembly 20. This feature also prevents the upper and lower brace parts 12, 14 from being pulled apart from each other.

In this embodiment, the constraining element 30, comprising the further recess 32 and further opening or channel 34, is provided in addition to the recess 22 and opening 24. Both features prevent the upper and lower brace parts 12, 14 from being pulled apart from each other when assembled using the upper and lower socket assemblies 20, 50. However, in alternative embodiments, the further opening or channel 34 could be used instead of the narrowed opening 24 of recess 22 for preventing the upper and lower brace parts 12, 14 from being pulled apart.

The dimensions of the further recess 32 of the constraining element 30 may be such that the outermost spring leaf 60a is fixed in position longitudinally relative to the socket assembly 20, i.e. the longitudinal dimension of the recess 32 may be matched to that of the end portion 64a or end cap 72 of the outermost spring leaf 60a. Alternatively, the further recess 32 may be dimensioned such that the outermost spring leaf 60a is able to slide longitudinally over a small distance relative to the socket assembly 20. For example, the recess 30 could be slightly longer than a longitudinal dimension of the end cap 72, for example up to 5mm longer to allow the outermost spring leaf 60a to slide longitudinally within the socket assembly 20 by up to 5mm. More preferably, longitudinal movement of the outermost spring leaf 60a is constrained to about 2mm or less. Nonetheless, the effect of the constraining element 30 is to limit longitudinal movement of the outermost spring leaf 60a with respect to longitudinal movement of the other spring leaves.

The constraining element 30 constrains the distance between the upper and lower brace parts 12, 14, while enabling the other spring leaves to slide relative to the outermost spring leaf 60a to allow the leaf spring hinge 16 to bend correctly. In other embodiments, alternative means may be provided for fixing or constraining the longitudinal position of the outermost spring leaf 60a within the socket assembly 20, for example a hook arranged to engage a corresponding hole or protrusion provided on the outermost spring leaf 60a.

As shown in Figure 2a, the outermost spring leaf 60a is longer than the other spring leaves 60. During bending, the individual spring leaves 60 slide relative to each other, with the outermost spring leaf 60a always having a larger radius of curvature than the other spring leaves 60. The spring leaves 60 may all have different lengths, decreasing progressively from the outermost spring leaf 60a (i.e. the forwardmost spring leaf) to the rearmost spring leaf, to take into account the change in relative position of the ends of the leaves 60 during bending. In this embodiment, the difference in length between the outermost spring leaf 60a and its adjacent spring leaf 60b is greater than a difference in length between said adjacent spring leaf 60b and the next adjacent spring leaf 60c. This extra distance between the end portion 64a of the outermost spring leaf 60a and the other spring leaves 60 enables the constraining element 30 to be positioned such that it does not obstruct the other spring leaves 60.

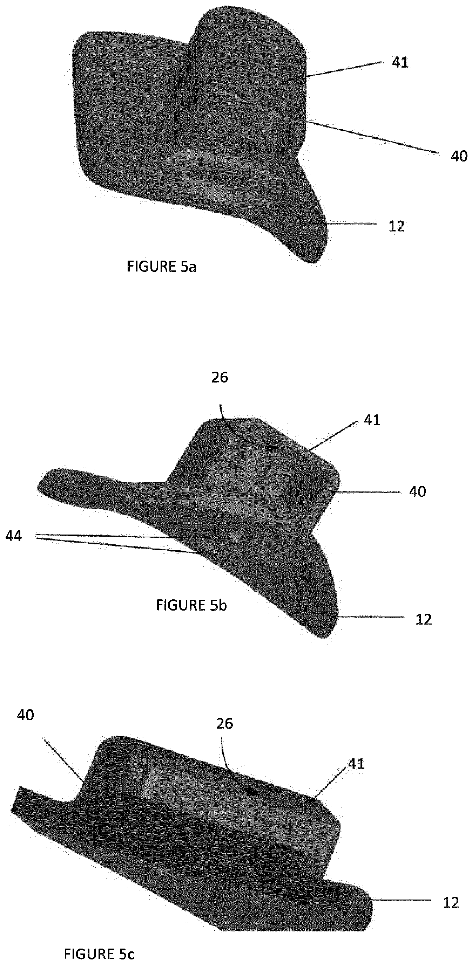

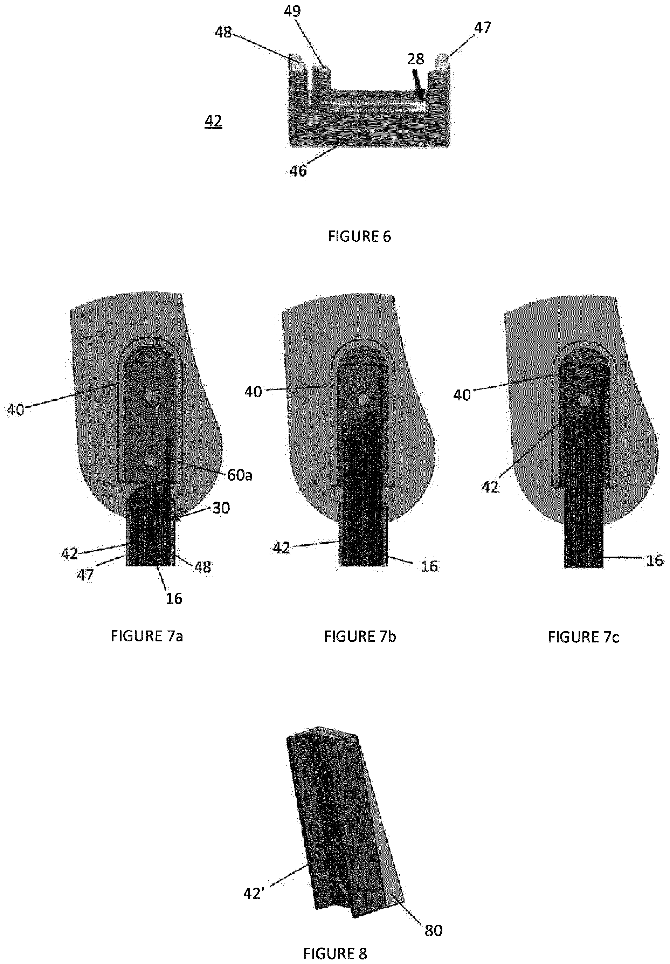

With reference to Figures 4a-h, 5a-c and 6, the first socket assembly 20 comprises a first socket part 40 and a second socket part 42, attached to each other such that the end portions 64 of the spring leaves 60 are received between opposing surfaces of the first and second parts 40, 42. In this embodiment, the first socket part 40, shown in Figures 5a-c, is integrally formed with the first or upper brace part 12. However, in other embodiments, the first socket part 40 may be formed as a separate part for attachment to one of the brace parts 12, 14. The second socket part 42, shown in Figure 6, is slidably receivable in said first socket part 40, preferably such that it does not protrude from the first socket part 40. In the present embodiment, holes 44, 45 are provided in the first and second socket parts 40, 42 respectively for receiving screws or other fastening elements for removably fixing the first and second socket parts 40, 42 together. The fastening elements are accessed from the limb-facing surface of the socket assembly 20 or brace part 12, so that the screw fastenings are not visible in use. The skilled person will appreciate that other fastening means may be used to attach the first and second socket parts 40, 42 together.

With reference to Figure 6, the second socket part 42 comprises a base 46, sidewalls 47, 48, substantially orthogonal to the base 46. A further wall 49, substantially parallel to the sidewalls 47, 48, is located between the side walls 47, 48. With reference to Figures 5a-c, the first socket part 40 is in the form of a sleeve, closed at one end, into which the second socket part 42 can be inserted. The first socket part 40 comprises an inner wall, against which the base 46 of the second part 42 is received, and an outer wall 41 which covers the second socket part 42, connected to the inner wall by sidewalls.

The recess 22 is bounded by the sidewalls 47, 48 of the second socket part 42 in the y-direction, and between the base 46 of the second socket part 42 and the outer wall 41 of the first socket part 40 in the x-direction. The outer wall 41 of the first socket part 40 is provided with a step 26 (Figures 4g, 4h) on its internal surface, so that the thickness of the outer wall 41 of the first socket part 40 increases between the recess 22 and the opening 24. The base 46 of the second socket part 42 is also provided with a step 28 (Figures 4f, 4g) so that the thickness of the base 46 increases between the recess 22 and the opening 24. The steps 26, 28 align when the first and second socket parts 40, 42 are assembled, so that the opening 24 is narrower than the recess 20 (in the x-direction) and prevents the end portions 64 of the spring leaves 60 from being pulled out of the recess 22.

The further recess 32 and further opening or channel 34 are bounded by opposing surfaces of the internal wall 49 and adjacent sidewall 48 of the second socket part 42 in the y-direction, and between the base 46 of the second socket part 42 and the outer wall 41 of the first socket part 40 in the x-direction. The opposing surfaces of the internal wall 49 and adjacent sidewall 48 of the second socket part 42 are provided with respective steps 49a, 48a (see Figures 4e and f), so that the respective thicknesses of the internal wall 49 and adjacent sidewall 48 of the second socket part 42 increase between the further recess 32 and the further opening or channel 34. The steps 48a, 49a are aligned so that the further opening 34 is narrower than the further recess 32 (in the y-direction) and prevents the end portion 64a of the outermost spring leaf 60a from being pulled out of the further recess 32.

Figures 7a-c illustrate a method for assembling the hinge assembly according to an embodiment of the invention. First, the second socket part 42 is arranged around the leaf spring hinge 16 such that the outermost spring leaf 60a is received in the channel 34 of the constraining element 30, and the remaining spring leaves 60 are received between the sidewalls 47, 48 of the recess 20, as shown in Figure 7a. Next, an end of the leaf spring hinge 16 is inserted into the first socket part 40 of the socket assembly 20, as shown in Figure 7b. Then, the second socket part 42 is pushed into the first socket part 40 such that the base 46 of the second socket part 42 is adjacent the inner wall of the first socket part 40 which, in use, is closest to a user, as shown in Figure 7c. This causes the end portion 64a of the outermost spring leaf 60a to be trapped in the further recess 32. The skilled person will appreciate that the second step can alternatively be interchanged with the first step, or combined with the third step. Screws (not shown) are inserted through holes 44 in the first socket part 40, from a user-facing side, into holes 45 in the second socket part 42. Each of the holes 45 of the second socket part 42 is designed to receive a nut to secure the screws. The holes 44 in the first socket part 40 are countersunk so that the screws do not protrude.

An alternative second socket part 42' according to a further embodiment is shown in Figure 8. The second socket part 42' is provided with a wedge portion 80 for adjusting a lateral angle of the first socket part 40 relative to the leaf spring hinge 16 (i.e. an angle about the y-axis defined relative to the leaf spring hinge 16). The wedge portion 80 is attached to or integrally formed with the second socket part 42'. Wedge portions 80 or second socket parts 42' providing different amounts of angle correction may be provided, or may be fabricated according to a user's needs. In this way, the lateral angle between the upper and lower brace parts 14, 16 may be adjusted by replacing the wedge portion 80 or second socket part 42' without replacing the entire hinge assembly or brace 10. A minimum amount of lateral angle correction may be included in the first socket part 40, with further correction provided by adjustment or replacement of the second socket part 42' as required. Correction of the lateral angle between the upper and lower brace parts 12, 14 in this way is only possible using a hard brace 10, since a conventional soft brace is not capable of maintaining a predefined angle between the end of the hinge 16 and a user's limb.

The skilled person will appreciate that the dimensions selected for the brace 10 and the leaf spring hinge 16 will depend on the size of the intended wearer. For a typical range of angle corrections, the opening 24 of the socket assembly may be approximately 10 mm, although this may be varied significantly, particularly if relatively large angle corrections are used.

A wide range of materials are suitable for the brace 10, including various plastics and composites such as nylon and polypropylene. The first and second socket parts 40, 42 of the socket assembly 20 may each be made using additive manufacturing techniques, which enables the parts to be lightweight and to be customised to a user if necessary. Additive manufacturing can be used to manufacture the first socket part 40 integrally with the upper brace part 12.

Although the invention has been described with reference to a specific embodiment of an unloader knee brace, the skilled person will appreciate that the hinge assembly of the present invention may be applied to braces for other joints. The skilled person will also appreciate that the hinge assembly could be incorporated into a soft brace, with each of the socket assemblies 20, 50 being attached to a soft sleeve.

It will be appreciated by persons skilled in the art that the above embodiments have been described by way of example only and not in any limitative sense, and that various alterations and modifications are possible without departure from the scope of the invention as defined by the appended claims.

## Claims

1. A hinge assembly for a brace (10) for supporting or guiding movement of a joint of a limb, the hinge assembly comprising:
a leaf spring hinge (16) comprising a plurality of spring leaves (60), wherein each spring leaf (60) comprises first and second end portions (64, 66) and a middle portion (68) extending longitudinally therebetween; and
a first socket assembly (20) comprising a recess (22, 32) for receiving respective first end portions (64) of the spring leaves (60; 60a) such that the middle portion (68) of at least one said spring leaf (60; 60a) extends through an opening (24; 34) in communication with said recess (22; 32);
wherein the first end portion (64; 64a) of said at least one spring leaf (60; 60a) has a transverse dimension (X2; Y2) greater than a corresponding transverse dimension (X1; Y1) of the middle portion (68) of the spring leaf (60; 60a), and wherein said opening (24; 34) has a transverse dimension (X3; Y3) which is smaller than the corresponding transverse dimension (X2; Y2) of said first end portion (64; 64a) of said at least one spring leaf (60; 60a).

2. A hinge assembly according to any of the preceding claims, wherein said first end portion (64) of said at least one spring leaf (60) comprises an end cap (72) attached to said spring leaf (60).

3. A hinge assembly according to claim 1 or claim 2, wherein the socket assembly (20) comprises a constraining element (30) for constraining longitudinal movement of the end portion (64a) of an outermost one (60a) of the plurality of spring leaves (60) relative to the socket assembly (20) with respect to the other spring leaves (60).

4. A hinge assembly according to claim 3, wherein said constraining element (30) is arranged to prevent longitudinal movement of the end portion (64a) of the outermost one (60a) of the plurality of spring leaves (60) relative to the socket assembly (20).

5. A hinge assembly according to claim 3 or claim 4, wherein said constraining element (30) comprises a channel (34) defined between two internal surfaces of said socket assembly (20) and arranged to receive the middle portion (68a) of said outermost one (60a) of said plurality of spring leaves (60), wherein said channel (34) has a transverse dimension (Y3) smaller than a corresponding transverse dimension (Y2) of said first end portion (64a) of said outermost spring leaf (60a).

6. A hinge assembly according to any one of claims 3 to 5, wherein the outermost spring leaf (60a) is longer than the other spring leaves (60).

7. A hinge assembly according to claim 6, wherein a difference in length between the outermost spring leaf (60a) and an adjacent spring leaf (60b) is greater than a difference in length between said adjacent spring leaf (60b) and the next adjacent spring leaf (60c).

8. A hinge assembly according to any of the preceding claims, wherein said first socket assembly (20) comprises:
a first socket part (40), fixable to or integrated into a part (12) of a brace (10) for attachment to a limb of a user; and
a second socket part (42) removably fixable to said first socket part (40);
wherein said end portion (64) of said at least one spring leaf (60) is receivable between opposing surfaces of said first and second socket parts (40, 42).

9. A hinge assembly according to claim 8, wherein said second socket part (42) is receivable in said first socket part (40).

10. A hinge assembly according to claim 9 and claim 5, wherein said channel (34) is defined between two surfaces of said second socket part (42).

11. A hinge assembly according to any of the preceding claims, wherein said socket assembly (20) comprises a wedge portion (80) for adjusting a lateral angle of a brace part (12) attached to or integrated to said socket assembly (20) relative to said leaf spring hinge (16).

12. A hinge assembly according to claim 11 and claim 9, wherein said wedge portion (80) is attached to or integrated into said second socket part (42).

13. A hinge assembly according to any of the preceding claims, further comprising:
a second socket assembly (50) comprising a second recess for receiving respective second end portions (66) of the spring leaves (60) such that the middle portion of at least one said spring leaf (60) extends through a second opening in communication with said second recess;
wherein the second end portion (66) of said at least one spring leaf (60) has a transverse dimension (X2; Y2) greater than a corresponding transverse dimension (X1; Y1) of the middle portion (68) of the spring leaf (60), and wherein said second opening has a transverse dimension which is smaller than the corresponding transverse dimension (X2; Y2) of said second end portion (66) of said at least one spring leaf (60).

14. A brace (10) for supporting or guiding movement of a joint of a limb, the brace (10) comprising:
an upper brace part (12) for attachment to the limb above the joint;
a lower brace part (14) for attachment to the limb below the joint; and
a hinge assembly according to any of the preceding claims, the hinge assembly connecting the upper and lower brace parts (12, 14);
wherein said first socket assembly (20) is fixed to one (12) of said upper and lower brace parts (12, 14).

15. A brace (10) according to claim 14 and claim 13, wherein said second socket assembly (50) is fixed to the other one (14) of said upper and lower brace parts (12, 14).
